# EUROPEAN PATENT APPLICATION

(11) **EP 4 252 553 A1**
(43) Date of publication of application: **04.10.2023**
(21) Application number: 21896236.3
(22) Date of filing: 28.05.2021
(51) Int. Cl.: A23L 33/135, A23L 33/26, A61K 35/747, A61P 1/00, A61P 37/04, A61K 31/715, A61K 31/702

(54) **APPLICATION OF LACTOBACILLUS PARACASEI SUBSP. PARACASEI K56 IN IMPROVING INTESTINAL BACTERIAL INFECTION RESISTANCE AND INTESTINAL IMMUNITY**

(30) Priority: 30.11.2020 CN 202011369266
(71) Applicant: Inner Mongolia Yili Industrial Group Co., Ltd., Inner Mongolia 010110 (CN); Inner Mongolia Dairy Tech Res Institute Co Ltd, Hohhot, Inner Mongolia 010110 (CN)
(72) Inventor: LIU, Wei-Hsien, Hohhot City, Inner Mongolia 010110 (CN); WEISS, Gisela Adrienne, Hohhot City, Inner Mongolia 010110 (CN); VAN LOO-BOUWMAN, Carolien Annika, Hohhot City, Inner Mongolia 010110 (CN); SMIT, Gerrit, Hohhot City, Inner Mongolia 010110 (CN); ZHAO, Wen, Hohhot City, Inner Mongolia 010110 (CN); HUNG, Wei-Lian, Hohhot City, Inner Mongolia 010110 (CN)
(74) Representative: Dehns
(86) International application number: PCT/CN2021/096955
(87) International publication number: WO 2022/110726

(57) **Abstract**

The present disclosure provides the use of *Lacticaseibacillus Paracasei* subsp. *paracasei* K56 in enhancing resistance of the intestinal tract to bacterial infection and improving intestinal immunity. Particularly, the present disclosure provides the use of a *Lacticaseibacillus Paracasei* subsp. *paracasei* in the preparation of a composition for improving intestinal immunity, wherein the *Lacticaseibacillus Paracasei* subsp. *paracasei* has a deposit number of CGMCC 15139 or DSM 27447. The present inventors have discovered that this bacteria strain alone can significantly improve the resistance of the intestinal tract to bacterial infection, combat invasion by pathogenic bacteria in the intestinal system, maintain the intestinal barrier function, and/or prevent diarrhea caused by pathogenic bacteria.

## Description

### Technical Field

The present disclosure relates to the technical field of microbiology, and particularly to a novel use of *Lacticaseibacillus Paracasei* subsp. *paracasei* K56 (deposit number CGMCC 15139 or DSM27447) in enhancing resistance of the intestinal tract to bacterial infection and improving intestinal immunity.

### Background

Intestinal epithelial cells are the first barrier of our body to pathogenic bacteria. The adhesion to or invasion of epithelial cells by pathogenic bacteria stimulates intestinal epithelial cells to produce inflammatory factors or chemokines. Some soluble mediators can modulate the intestinal immune system or induce cellular innate immune responses. Adequate immune responses and a good barrier function of epithelial cells would help protect the host from infection by pathogenic bacteria.

Probiotics refer to a class of living microorganisms that confer health benefits to the human body when ingested in a sufficient amount. As an important component of the intestinal microbiome, probiotics play an unneglectable role in human health, such as nutrient supply, synthesis of vitamins, aid in digestion, promoting angiogenesis and enteric nerve function.

### Summary of the Invention

An objective of the present disclosure is to provide a novel use of *Lacticaseibacillus Paracasei* subsp. *paracasei* K56.

The present disclosure provides a novel use of a *Lacticaseibacillus Paracasei subsp. Paracasei* strain, which is designated as K56 herein. This bacterial strain was deposited in the German Collection of Microorganisms and Cell Cultures on June 27, 2013 with deposit number DSM27447. In addition, the *Lacticaseibacillus Paracasei subsp. paracasei* K56 strain was also deposited in the China General Microbiological Culture Collection Center (CGMCC) on December 29, 2017 with deposit number CGMCC 15139.

In the present disclosure, *Lacticaseibacillus Paracasei subsp. paracasei* K56 was studied for its function in improving intestinal immunity by using an enterotoxigenic *Escherichia coli* strain ETEC H10407 and an enteropathogenic *Escherichia coli* strain EPEC 0119 as pathogenic bacterial strains that induce intestinal infection *in vitro.* Both *E. coli* strains are common pathogenic bacteria, causing infantile diarrhea and traveler's diarrhea in developing countries or areas of poor sanitation. ETEC H10407 is a well-established model strain commonly used in experiments, and is often used to evaluate the ability of probiotics to adhere to pathogenic bacteria and inflammatory signaling pathways.

It has been discovered in the present disclosure that the *Lacticaseibacillus Paracasei subsp. paracasei* K56 strain (i.e., *Lacticaseibacillus Paracasei subsp. paracasei* with deposit number CGMCC 15139 or DSM27447) has an effect of inhibiting adhesion by intestinal pathogenic bacteria, in that it can significantly inhibit adhesion by ETEC H10407 and EPEC 0119 to Caco-2 cells and improve the integrity of the intestinal barrier, and also has an effect of inhibiting production of intestinal inflammatory factors, in that it can mitigate inflammatory responses caused by ETEC H10407.

As such, in one aspect, the present disclosure provides the use of a *Lacticaseibacillus Paracasei subsp. Paracasei* strain in the preparation of a composition for improving intestinal immunity, wherein the *Lacticaseibacillus Paracasei* subsp. *paracasei* strain has a deposit number of CGMCC 15139 or DSM 27447.

According to an embodiment of the present disclosure, for the use of the present disclosure, the *Lacticaseibacillus Paracasei subsp. Paracasei* strain is used in a form of a solid or liquid formulation of live bacteria and/or dead bacteria for the preparation of the composition.

According to an embodiment of the present disclosure, for the use of the present disclosure, the improving intestinal immunity includes: improving resistance of the intestinal tract to bacterial infection, combating invasion by pathogenic bacteria in the intestinal system, maintaining the intestinal barrier function, and/or preventing diarrhea caused by pathogenic bacteria.

According to an embodiment of the present disclosure, for the use of the present disclosure, the improving intestinal immunity includes: lowering the ability of pathogenic bacteria to adhere to intestinal epithelial cells, and/or reducing the release of the inflammatory factor IP-10 from intestinal cells caused by pathogenic bacteria.

According to an embodiment of the present disclosure, for the use of the present disclosure, the *Lacticaseibacillus Paracasei* subsp. *paracasei* strain is administered in an amount of 1.0×10³ to 1.0×10¹² CFU/day, or 0.001 µg to 1000 mg per day of the weight of the bacteria strain. Preferably, the *Lacticaseibacillus Paracasei* subsp. *paracasei* strain is administered in an amount of 10⁶ to 10¹¹ CFU/day, or 1 µg to 10 mg per day of the weight of the bacteria strain.

According to an embodiment of the present disclosure, the composition may comprise a food composition, a feed composition, or a pharmaceutical composition.

According to an embodiment of the present disclosure, the composition may be administered to an animal or human. The composition may also include ingredients conventionally used in the art. For example, for a pharmaceutical composition, an appropriate amount of adjuvants may be included, and the adjuvants may be excipients, diluents, fillers, absorption enhancers, and the like. For a food composition, the *Lacticaseibacillus Paracasei* subsp. *paracasei* strain of the present disclosure can be produced in accordance with a food product in the art containing *Lacticaseibacillus Paracasei* subsp. *paracasei,* and the composition may be in various forms according to the needs of the recipient, for example, powder, lozenges, granules, microcapsules, a liquid formulation, and the like.

In an embodiment of the present disclosure, the composition is a food composition, and the food may be a liquid beverage, a solid beverage, an oral liquid, a diary product, a tablet, or a capsule; for example, it may be a fermented milk product (such as fermented milk, flavored fermented milk, fermented milk beverage, etc.), cheese, a milk-containing beverage, a probiotic solid beverage, milk powder or the like.

In another embodiment of the present disclosure, the composition is a feed composition. The other components in the feed composition may be selected with reference to conventional techniques in the field of probiotic feeds.

In another embodiment of the present disclosure, the composition is a pharmaceutical composition. The other components in the pharmaceutical composition may be selected with reference to conventional techniques in the field of probiotic medicaments.

In another aspect, the present disclosure further provides a method for improving intestinal immunity, the method comprising:
administering to a subject an effective amount of a *Lacticaseibacillus Paracasei* subsp. *paracasei* strain to improve intestinal immunity; wherein the *Lacticaseibacillus Paracasei* subsp. *paracasei* strain has a deposit number of CGMCC 15139 or DSM27447.

Furthermore, the present disclosure provides *Lacticaseibacillus Paracasei* subsp. *paracasei* K56 for use in improving intestinal immunity.

According to an embodiment of the present disclosure, in the method of the present disclosure, the improving intestinal immunity includes: improving the resistance of the intestinal tract to bacterial infection, combating invasion by pathogenic bacteria in the intestinal system, maintaining the intestinal barrier function, preventing diarrhea caused by pathogenic bacteria, lowering the ability of pathogenic bacteria to adhere to intestinal epithelial cells, and/or reducing the release of the inflammatory factor IL-8 and/or IP-10 from intestinal cells induced by pathogenic bacteria.

According to an embodiment of the present disclosure, in the method of the present disclosure, the *Lacticaseibacillus Paracasei* subsp. *paracasei* strain is administered in an amount of 1.0×10³ to 1.0×10¹² CFU/day, or 0.001 µg to 1000 mg per day of the weight of the bacteria strain;
preferably, the *Lacticaseibacillus Paracasei* subsp. *paracasei* strain is administered in an amount of 10⁶ to 10¹¹ CFU/day, or 1 µg to 100 mg per day of the weight of the bacteria strain.

In summary, the present disclosure provides a novel use of *Lacticaseibacillus Paracasei* subsp. *paracasei* K56, which has effects of significantly reducing the ability of pathogenic bacteria to adhere to intestinal epithelial cells, improving the intestinal barrier function, and activating intestinal immunity. This bacteria strain may be used in the preparation of a food, medicament, or feed product capable of anti-infection and modulating intestinal health and immunity, and may find broad applications.

### Description of Drawings

Fig. 1 shows a curve of viable bacteria counts of *Lacticaseibacillus Paracasei* subsp. *paracasei* K56.
Fig. 2 shows the effect of *Lacticaseibacillus Paracasei* subsp. *paracasei* K56 in inhibiting adhesion by pathogenic *Escherichia coli* EPEC 0119 to Caco-2 (p<0.001).
Fig. 3 shows the changes in TEER (Ω.cm²) after co-culturing of *Lacticaseibacillus Paracasei* subsp. *paracasei* K56 and ETEC H10407 for 1 h, 2 h, 3 h, and 4 h.
Figs. 4A and 4B show the effect of *Lacticaseibacillus Paracasei* subsp. *paracasei* K56 on the expression of the pro-inflammatory factors (IL-8, IP10) in Caco-2 cells (without ETEC H10407).
Figs. 5A and 5B show the effect of *Lacticaseibacillus Paracasei* subsp. *paracasei* K56 on the inflammatory factors (IL-8, IP10) in Caco-2 cells.

### Detailed Description of the Invention

In order to provide a better understanding of the technical features, purposes and advantageous effects of the present disclosure, the technical solutions of the present disclosure are described in details hereinafter in connection with specific examples, and it should be understood that these examples are merely illustrative of the present disclosure and not to limit the scope of the present disclosure. In the Examples, the experimental methods, where no specific conditions are indicated, are conventional methods and conditions well known in the art, or are performed as recommended by the instrument manufacturer.

### Viable bacteria counts of Lacticaseibacillus Paracasei subsp. paracasei K56 used in the Examples:

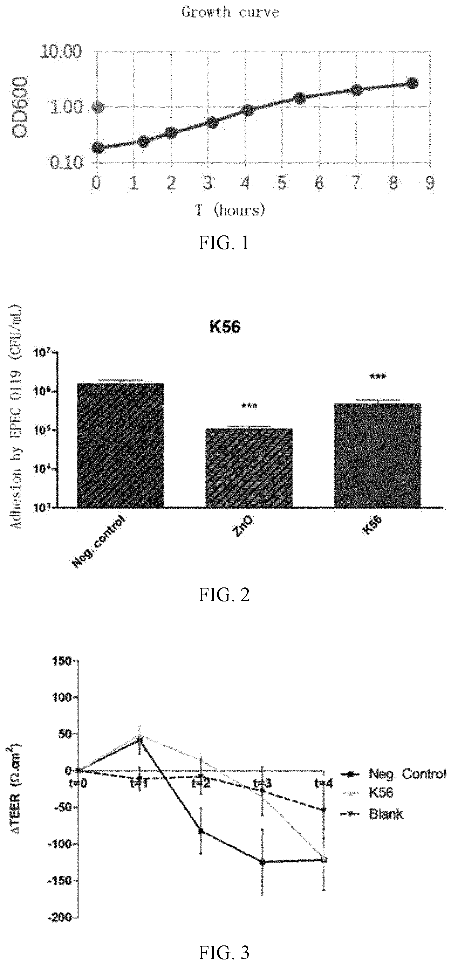

A single colony of *Lacticaseibacillus Paracasei* subsp. *paracasei* K56 (deposit number CGMCC 15139 or DSM27447) was picked from a spread plate, and after 16S identification, stored in a glycerin tube at -80°C. Before the experiment, the strain was cultured in an MRS medium at 37°C, grown till the logarithmic phase, and enriched by centrifugation. The number of viable bacteria and bacterial activity were measured by fluorescence activated cell sorting (FACS), and the results are shown in Fig. 1. The strain reached a growth plateau at 8 h, and the number of viable bacteria became stable, and stabilized around 10⁸ cfu/mL at 8 h. The viable bacteria counts of the strain used in the Examples of the present disclosure were 1×10⁸ CFU/mL or 1×10⁶ CFU/mL.

### Data analysis in the Examples:

All data in the experiment were obtained at least in triplicate, and the experimental results were expressed as mean ± standard error. Data analysis was performed by using SPSS17.0 software (SPSS, Chicago, IL, USA), and the significance analysis was performed by one-way analysis of variance by the LSD method. P-value<0.05 is considered as a statistically significant difference, and the significant difference is categorized as follows: *p<0.05, **p<0.01, ***p<0.001, ****p<0.0001. Figures were drawn with GraphPad Prism 5.0.

### Example 1. Determination of anti-adhesion ability

A co-culturing method was performed to investigate whether *Lacticaseibacillus Paracasei* subsp. *paracasei* K56 inhibits EPEC 0119 from adhering to Caco-2. Caco-2 cells were cultured in a 24-well plate, washed with a PBS buffer, and co-cultured with the strain for 1 h. EPEC O119 was added to the Caco-2 cells at a multiplicity of infection (MOI) of 50:1, and co-cultured for 1 h (37°C), to a final viable bacteria count of 1×10⁷ CFU/mL. A group of Caco-2 without addition of *Lacticaseibacillus Paracasei* subsp. *paracasei* K56 was used as a negative control. As a positive control group, 1 mM zinc oxide (ZnO) was used instead of *Lacticaseibacillus Paracasei* subsp. *paracasei* K56. After incubation, the Caco-2 cells were washed and lysed, and the pathogenic bacteria were inoculated on agar. After culturing on the agar plate at 37°C overnight, the number of colonies of the bacteria in CFU was counted to measure the adhesion by the pathogenic bacteria. The number of colonies of growing *E. coli* was counted and recorded as CFU/mL. In parallel to the anti-adhesion test, *E. coli* of 10⁷ CFU/mL (final concentration) was added to 1 mL of the test substance (K56 strain solution) and co-cultured at 37°C for 1 hour to measure the activity. After the culturing, *E. coli* was collected from each sample by centrifugation, resuspended in PBS, and inoculated on an agar plate. After culturing on the agar plate at 37°C overnight, the number of colonies of the bacteria in CFU was counted to measure the adhesion by the pathogenic bacteria. The number of colonies of growing *E. coli* was counted and recorded as CFU/mL.

With the human intestinal cells Caco-2 described above as a model, it is confirmed in the present disclosure that *Lacticaseibacillus Paracasei* subsp. *paracasei* K56 has an inhibitory effect on the enteropathogenic *Escherichia coli* strain EPEC 0119.

In some experiments of the present disclosure, the experimental results of the ability of *Lacticaseibacillus Paracasei* subsp. *paracasei* K56 at a concentration of 10⁸ CFU/mL to inhibit EPEC 0119 are shown in Fig. 2. Without intervention with *Lacticaseibacillus Paracasei* subsp. *paracasei* K56, the number of the enteropathogenic *E. coli* strain EPEC 0119 adhered to Caco-2 cells was as high as 1.73×10⁶ CFU/mL (6.23 log CFU/mL). When the enteropathogenic *E*. *coli* EPEC 0119 was present together with *Lacticaseibacillus Paracasei* subsp. *paracasei* K56, the number of the adhered enteropathogenic *E. coli* EPEC 0119 was 4.83×10⁵ CFU/mL (5.68 log CFU/mL), indicating 72.08% inhibition. The experimental results indicate that *Lacticaseibacillus Paracasei* subsp. *paracasei* K56 has a significant inhibitory effect on the enteropathogenic *Escherichia coli* strain EPEC 0119 (p<0.001).

In other experiments of the present disclosure, the inhibitory effects of *Lacticaseibacillus Paracasei* subsp. *paracasei* K56 at two different concentrations of 10⁶ CFU/mL and 10⁸ CFU/mL on the adhesion by *Escherichia coli* EPEC 0119 to Caco-2 cells were tested in two experiments. The data of the experimental results (mean ± standard error, from triplicate tests) are shown in Table 1. Upon comparison of the negative control with the test groups by Dunnett's post-hoc test, it was found that in the two experiments, K56 at a concentration of 10⁶ significantly reduced the pathogenic adhesion as compared to the negative control (p<0.05), and K56 at a concentration of 10⁸ also significantly reduced the adhesion by the pathogenic bacteria (p<0.05).

**Table 1**

| Groups | Adhered EPEC 0119 (CFU/mL) | |
|---|---|---|
| | Mean | Standard error |
| Negative control, experiment 1 | 1400000 | 104083 |
| K56 (10⁶) | 733333 | 130171 |
| | | |
| Negative control, experiment 2 | 1706250 | 200988 |
| K56 (10⁸) | 683333 | 44096 |

The above experimental results indicate that *Lacticaseibacillus Paracasei* subsp. *paracasei* K56 has an inhibitory effect on the enteropathogenic *Escherichia coli* strain EPEC 0119.

### Example 2. Intestinal barrier integrity

An ideal intestinal epithelial barrier function is a prerequisite for protecting the host from pathogenic bacteria invasion and/or pathogen-derived toxins. In this study, *in vitro* barrier integrity was evaluated by measurement of the transepithelial/transendothelial electrical resistance (TEER) of intestinal cell layers. In order to study the effect of probiotics on infection, the change in TEER over time before and after *E. coli* infection was measured.

Caco-2 cells were seeded at a concentration of 2×10⁴ cells/cm² into Transwell polycarbonate cell culture inserts with an average pore size of 0.4 µm and an area of 0.33 cm², until complete differentiation (±1000Ω). Transepithelial/transendothelial electrical resistance (TEER) was measured with an EVOM2 epidermal voltmeter purchased from World Precision Instruments to measure the barrier integrity.

On the day of test, cells were washed and incubated for 1 h at 37°C in a medium free of antibiotics and serum but containing a test substance. *Escherichia coli* was then added to the test substance (at an MOI of 200:1) and incubated for 6 h. TEER was measured before the start of the experiment (t=-1), 1 h after exposure to the test substance before addition of the pathogenic bacteria (t=0), and 1 h, 2 h, 3 h, 4 h, 6 h after exposure to the pathogenic bacteria, respectively. The TEER values measured under individual conditions after exposure to the pathogenic bacteria were correlated to their respective TEER values at t=0, and were expressed as ΔTEER (Ω.cm²). Negative controls (with addition of *E. coli* only) and positive controls (not exposed to the pathogenic bacteria or test substances) were also included in the experiments. All conditions were tested in triplicate, and some control groups were tested in six replicates.

After challenge with ETEC H10407 (MOI=50) for 6 hours, the blank control group showed a poor TEER value, indicating that the survival rate of the cells decreased drastically after 6 h challenge. Thus, it was preliminarily determined that the challenge duration with ETEC H10407 should be kept within 4 h.

Fig. 3 shows the changes in TEER (Ω.cm²) after co-culturing of *Lacticaseibacillus Paracasei* subsp. *paracasei* K56 at a concentration of 10⁸ CFU/mL and ETEC H10407 for 1 h, 2 h, 3 h, and 4 h. As seen from Fig. 3, after K56 was added, TEER increased significantly (p<0.001). After treatment for 4 h, TEER of the K56 group decreased in a time-dependent manner, and at t=4 h, there was still a significant difference from the ETEC H10407 group (p<0.01). This suggests that the intervention with *Lacticaseibacillus Paracasei* subsp. *paracasei* K56 significantly mitigated the damage caused by ETEC H10407 to the integrity of the intestinal cell barrier.

In other experiments of the present disclosure, the effects of *Lacticaseibacillus Paracasei* subsp. *paracasei* K56 at two different concentrations of 10⁶ CFU/mL and 10⁸ CFU/mL on the transepithelial/transendothelial electrical resistance (TEER) after exposure to *E. coli* ETEC H10407 were tested. The results are shown in Tables 2 and 3. Experimental data were measured in triplicates.

**Table 2**

| Time | Negative control | | Blank control | | K56 (10⁸) | |
|---|---|---|---|---|---|---|
| | Mean | Standard error | Mean | Standard error | Mean | Standard error |
| t=0 | 0 | 0 | 0 | 0 | 0 | 0 |
| t=1 | 19.7 | 3.0 | 15 | 4.6 | 44 | 6.1 |
| t=2 | 0.3 | 2.2 | 17.7 | 6.9 | 35.7 | 2.3 |
| t=4 | -19.3 | 1.5 | 12.7 | 11.2 | 4.3 | 3.3 |
| t=6 | -54 | 4.0 | -2.7 | 13.4 | -26.7 | 4.1 |

**Table 3**

| Time | Negative control | | Blank control | | K56 (10⁶) | |
|---|---|---|---|---|---|---|
| | Mean | Standard error | Mean | Standard error | Mean | Standard error |
| t=0 | 0 | 0 | 0 | 0 | 0 | 0 |
| t=1 | 39.7 | 2.4 | 10.3 | 5.5 | 26 | 7.2 |
| t=2 | -21 | 2.6 | 13 | 8.1 | -9.7 | 7.2 |
| t=4 | -80.7 | 3.3 | -2.3 | 7.2 | -100 | 6.7 |
| t=6 | -116.3 | 7.5 | -1.7 | 7.0 | -155.7 | 14.7 |

Compared with the negative control, *Lacticaseibacillus Paracasei* subsp. *paracasei* K56 at the concentration of 10⁶ CFU/mL and 10⁸ CFU/mL tend to increase the TEER values at various time points.

### Example 3. Determination of immunomodulatory ability

The immunomodulatory ability of *Lacticaseibacillus Paracasei* subsp. *paracasei* K56 was evaluated based on whether it can reduce the production of IL-8 and IP-10 in Caco-2 cells induced by ETCT H10407. Caco-2 cells were cultured in a 96-well plate to an appropriate abundance. At the beginning of the experiment, the cells were washed once with an antibiotic-free medium. A monolayer of cells was co-cultured with a test substance in an antibiotic-free medium for 1 h at 37°C, which was repeated three times. *E. coli* was added to stimulate the cells (MOI 200: 1). After 1 hour of incubation, the cell monolayer was co-cultured with the pathogenic bacteria, washed and incubated overnight with a medium containing a test substance and 50 µg/mL gentamicin,. For a blank control (Blank), only the medium was used, without *E. coli* stimulation. A medium stimulated with *E. coli* but without a test substance was used as a control for the *E. coli* response. In addition, as a control for the response of Caco-2 cells, the cells were stimulated with a medium containing a mixture of Rec TNFα (10 ng/mL) and Rec IFNγ (5 ng/mL) (both purchased from R&D Systems, Abingdon, UK). 24 hours after stimulation, supernatant was collected and stored at -20°C. IL-8 and IP-10 were tested with a Bio-Plex kit (BioRad, California, USA) according to the manufacturer's instructions.

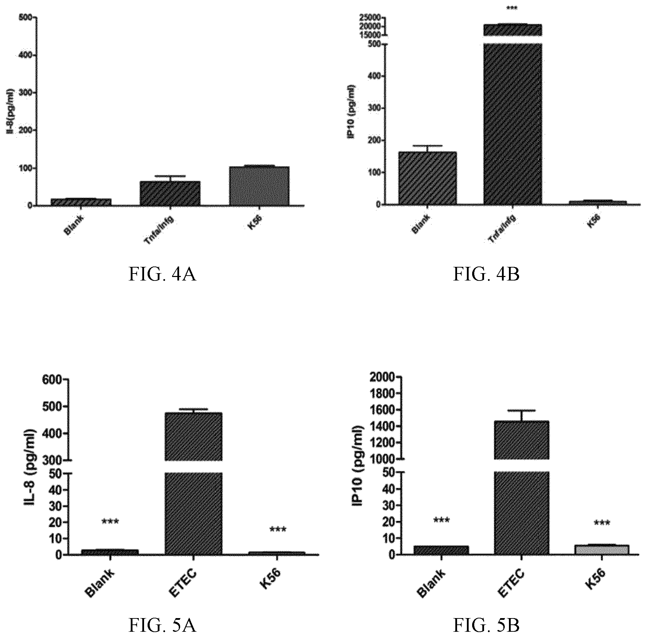

The experimental results for *Lacticaseibacillus Paracasei* subsp. *paracasei* K56 at a concentration of 10⁸ CFU/mL are shown in Figs. 4A, 4B, 5A, and 5B. As seen from Figs. 4A and 4B, *Lacticaseibacillus Paracasei* subsp. *paracasei* K56 had no significant effect on the expression of the pro-inflammatory factors IL-8 and IP10 in Caco-2 when ETEC H10407 was not added (p>0.05). As seen from Figs. 5A and 5B, ETEC H10407 can induce a pro-inflammatory response in the intestinal epithelial cells Caco-2, and addition of ETEC resulted in a significant upregulation of IL-8 and IP 10 in the Caco-2 cell model. After the intervention with ETEC H10407, the IL-8 content in the cell culture increased from 2.62 ± 0.44 pg/mL to 473.84 ± 21.9 pg/mL, and after the K56 treatment, the expression of IL-8 returned to 1.33 ± 0.19 pg/mL, indicating that *Lacticaseibacillus Paracasei* subsp. *paracasei* K56 has a significant inhibitory effect on the increase in IL-8 induced by ETEC H10407 (p<0.001). The IP10 content in the cell culture increased from 5 ± 0.00 pg/mL to 1454.08 ± 193.31 pg/mL, and after the K56 treatment, the expression of IP10 returned to 5.54 ± 0.76 pg/mL, indicating that *Lacticaseibacillus Paracasei* subsp. *paracasei* K56 has a significant inhibitory effect on the increase in IP10 induced by ETEC H10407 (p<0.001). The test results indicate that *Lacticaseibacillus Paracasei* subsp. *paracasei* K56 inhibits the production of inflammatory factors IL-8 and IP10 and significantly inhibits the inflammatory responses caused by ETEC 10407 (p<0.001), which further indicates that K56 has a potential for reducing the inflammatory responses caused by ETEC 10407 to a certain extent.

As confirmed from the above research results, *Lacticaseibacillus Paracasei* subsp. *paracasei* K56 can significantly inhibit the adhesion by ETEC H1407 and EPEC 0119 to Caco-2 cells, and alleviate the inflammatory responses caused by ETEC H10407, indicating that *Lacticaseibacillus Paracasei* subsp. *paracasei* K56 has a good potential for preventing infant diarrhea, traveler's diarrhea, intestinal inflammation and the like.

## Claims

1. Use of a *Lacticaseibacillus Paracasei* subsp. *paracasei* strain in the preparation of a composition for improving intestinal immunity, wherein the *Lacticaseibacillus Paracasei* subsp. *paracasei* strain has a deposit number of CGMCC 15139 or DSM 27447.

2. The use according to claim 1, wherein the *Lacticaseibacillus Paracasei* subsp. *paracasei* strain is used in a form of a solid or liquid formulation of live bacteria and/or dead bacteria for the preparation of the composition.

3. The use according to claim 1, wherein the improving intestinal immunity includes: improving the resistance of the intestinal tract to bacterial infection, combating invasion by pathogenic bacteria in the intestinal system, maintaining the intestinal barrier function, and/or preventing diarrhea caused by pathogenic bacteria.

4. The use according to claim 1, wherein the improving intestinal immunity includes: lowering the ability of pathogenic bacteria to adhere to intestinal epithelial cells, and/or reducing the release of the inflammatory factor IL-8 and/or IP-10 from intestinal cells induced by pathogenic bacteria.

5. The use according to claim 3 or 4, wherein the pathogenic bacteria are *Escherichia coli.*

6. The use according to claim 5, wherein the *Escherichia coli* includes the enterotoxigenic *Escherichia coli* strain ETEC H10407 and/or the pathogenic *Escherichia coli* strain EPEC 0119.

7. The use according to claim 3 or 4, wherein the *Lacticaseibacillus Paracasei* subsp. *paracasei* strain is administered in an amount of 1.0×10³ to 1.0×10¹² CFU per day, or 0.001 µg to 1000 mg per day of the weight of the bacteria strain.

8. The use according to claim 7, wherein the *Lacticaseibacillus Paracasei* subsp. *paracasei* strain is administered in an amount of 10⁶ to 10¹¹ CFU per day, or 1 µg to 100 mg per day of the weight of the bacteria strain.

9. The use according to claim 1, wherein the composition includes a food composition, a feed composition, or a pharmaceutical composition.

10. The use according to claim 9, wherein the food is a liquid beverage, a solid beverage, an oral liquid, a diary product, a tablet, or a capsule.

11. A method for improving intestinal immunity, the method comprising:
administering to a subject an effective amount of a *Lacticaseibacillus Paracasei* subsp. *paracasei* strain to improve intestinal immunity, wherein the *Lacticaseibacillus Paracasei* subsp. *paracasei* strain has a deposit number of CGMCC 15139 or DSM 27447.

12. The method according to claim 11, wherein the improving intestinal immunity includes: improving the resistance of the intestinal tract to bacterial infection, combating invasion by pathogenic bacteria in the intestinal system, maintaining the intestinal barrier function, preventing diarrhea caused by pathogenic bacteria, lowering the ability of pathogenic bacteria to adhere to intestinal epithelial cells, and/or reducing the release of the inflammatory factor IL-8 and/or IP-10 from intestinal cells induced by pathogenic bacteria.

13. The method according to claim 11 or 12, wherein the *Lacticaseibacillus Paracasei* subsp. *paracasei* strain is administered in an amount of 1.0×10³ to 1.0×10¹² CFU per day, or 0.001 µg to 1000 mg per day of the weight of the bacteria strain;
preferably, wherein the *Lacticaseibacillus Paracasei* subsp. *paracasei* strain is administered in an amount of 10⁶ to 10¹¹ CFU per day, or 1 µg to 100 mg per day of the weight of the bacteria strain.
